# FASCICULE DE BREVET EUROPEEN

(11) **EP 0 881 901 B1**
(45) Date de publication et mention de la délivrance du brevet: **12.06.2002**
(21) Numéro de dépôt: 97905218.0
(22) Date de dépôt: 17.02.1997
(51) Int. Cl.: A61K 31/60, A61P 7/02

(54) **NOUVELLES ASSOCIATIONS DE PRINCIPES ACTIFS CONTENANT DU CLOPIDOGREL ET UN ANTITHROMBOTIQUE**
VERBINDUNGEN VON WIRKSTOFFEN, WELCHE KLOPIDOGREL UND ANTITHROMBOTISCHE MITTEL ENTHALTEN
NEW ASSOCIATIONS OF ACTIVE PRINCIPLES CONTAINING CLOPIDOGREL AND AN ANTITHROMBOTIC AGENT

(30) Priorité: 19.02.1996 FR 9602027
(43) Date de publication de la demande: 09.12.1998
(73) Titulaire: SANOFI-SYNTHELABO, 75635 Paris Cédex 13 (FR)
(72) Inventeur: BERNAT, André, F-31270 Cugnaux (FR); HERBERT, Jean-Marc, F-31170 Tournefeuille (FR); SAVI, Pierre, F-31600 Muret (FR)
(74) Mandataire: Bernasconi, Jean Raymond
(86) Numéro de dépôt international: FR9700296
(87) Numéro de publication internationale: WO9729753

(56) Documents cités:
- FR-A- 2 307 538
- EXPERT OPIN. INVEST. DRUGS, 1994, 3/5 (449-455), UNITED KINGDOM, XP000610730 HERBERT J.-M.: "Clopidogrel and antiplatelet therapy"

## Description

La présente invention a pour objet une nouvelle association de principes actifs à activité anti-agrégante plaquettaire constituée de clopidogrel et d'aspirine, et les compositions pharmaceutiques les contenant.

Les principes actifs constituant l'association sont présents à l'état libre ou sous forme d'un de leurs sels pharmacologiquement acceptable.

Au cours de cette dernière décennie, un grand intérêt a été accordé à l'étude du rôle joué par les plaquettes dans le développement des maladies associées à l'athérosclérose (infarctus du myocarde, angor, attaque cérébrale, maladies artérielles périphériques....). Le rôle bien établi des plaquettes dans la thrombose artérielle a permis le développement de nombreux médicaments qui inhibent les fonctions des plaquettes et la découverte du rôle essentiel de l'ADP dans le processus thrombotique a conduit au développement de la ticlopidine, un puissant agent antithrombotique. Ce dérivé de thiéno [3,2-c]pyridine est décrit dans le brevet FR 73 03503. La ticlopidine inhibe sélectivement l'agrégation plaquettaire induite par l'ADP ainsi que celle d'autres agonistes, médiée par l'ADP. (Féliste et al., Thromb. Res., 1987, *48*, 403-415).

Dans des études cliniques multicentriques en double-aveugle, la ticlopidine s'est révélée être significativement plus efficace que l'aspirine ou qu'un placebo dans la prévention de l'attaque cérébrale chez des patients présentant un haut risque d'accidents vasculaires (Gent et al., Lancet, 1989, *8649*, 1215-1220 ; Hass et al., N. Engl. J. Med., 1989, *321*, 501-507). Elle s'est également avéré significativement plus efficace que le placebo chez des patients présentant un fort risque d'accidents vasculaires centraux et périphériques ( Janzon et al., Scand. J. Int. Med., 1990, *227*, 301-308).

Bien qu'il soit connu, à ce jour, que l'aspirine et la ticlopidine agissent via deux mécanismes d'action différents, de nombreuses études ont comparé l'efficacité de ces deux médicaments et c'est seulement très récemment que quelques études ont suggéré que la ticlopidine administrée en association avec l'aspirine pourrait être d'un grand intérêt vis à vis de la thrombose aiguë, en remplacement des traitements actuels peu efficaces, chez des patients auxquels avaient été implantés des prothèses endovasculaires métalliques (Van Belle et al., Cor. Art. Dis., 1995, *6*, 341-345).

L'association de la ticlopidine et de l'aspirine est revendiquée dans le brevet FR 75 12084 pour son utilisation en tant qu'antiagrégant plaquettaire pourvu d'un effet hémodynamique nettement supérieur, qualitativement et quantitativement, à celui de la ticlopidine seule. Ces résultats ont été mis en évidence à l'aide d'études pharmacologiques qui ont porté sur les propriétés inhibitrices de l'agrégation plaquettaire en effectuant des mesures de l'agrégation plaquettaire induite par l'ADP ou le collagène. Les résultats qui ont été obtenus sont prédictifs d'un intérêt thérapeutique de l'association ticlopidine-aspirine dans certains types de thromboses aiguës consécutives notamment à certaines interventions chirurgicales mais ne suffisent pas pour en déduire une indication dans la prévention secondaire des accidents vasculaires dans la maladie athéromateuse ou encore dans l'endartérectomie ou pose de prothèses endovasculaires métalliques.

Il est par ailleurs connu que d'autres associations d'anti-agrégants plaquettaires telles que par exemple l'association aspirine-dipyridamole ont fait l'objet d'études cliniques versus dipyridamole seul ou aspirine seule dans l'étude de la prévention de l'accident vasculaire cérébral ou de l'occlusion du shunt vasculaire chez des patients. La conclusion de ces études a été que l'association aspirine-dipyridamole ne possède aucun effet bénéfique significativement plus important que celui observé avec le dipyridamole seul ou l'aspirine seule dans la prévention secondaire de l'ischémie athérothrombotique cérébrale ou vis à vis de la thrombose (Acta. Neurol. Scand., 1987, *76(6),* 413-421 ; Thrombosis, 1994, Alert n°12 ; Thrombosis, 1994, Alert n°9 ; Thrombosis, 1993, Alert n°9 ; Thrombosis, 1993, Alert n°2).

La pose de prothèses endovasculaires métalliques au niveau coronarien et carotidien peut être considérée aujourd'hui comme un important progrès thérapeutique dans la prévention et le traitement des accidents vasculaires centraux et périphériques. Néanmoins ces prothèses présentent un puissant effet pro-thrombotique du à leur nature métallique qu'il est aujourd'hui primordial de prévenir à l'aide d'agents anti-thrombotiques et principalement anti-agrégants plaquettaires.

Un autre dérivé de thiénopyridine, le clopidogrel décrit dans EP 099 802 s'est également révélé être un puissant antithrombotique, agissant selon un mécanisme d'action identique à celui de la ticlopidine (Savi et al., J. Pharmacol. Exp. Ther., 1994, *269,* 772-777 ; Herbert et al., Cardiovasc. Drug Rev., 1993, *11,* 180-198).

Son utilisation serait bénéfique vis à vis d'états pathologiques tels que les troubles du système cardio-vasculaire et cérébrovasculaire comme les troubles thromboemboliques associés à l'athérosclérose ou au diabète tels l'angine instable, l'attaque cérébrale, la resténose après angioplastie, l'endartérectomie ou pose de prothèses endovasculaires métalliques, à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires, de pontages aorto coronariens ou vis à vis de l'angor stable ou instable.

Le clopidogrel est, selon les agents agrégants utilisés, chez l'animal et chez l'homme environ 10 à 50 fois plus efficace que la ticlopidine. De plus, à la différence de celle-ci, le clopidogrel présente une activité antiagrégante quasi immédiate, apparaissant dans les 15 minutes après l'administration alors que la ticlopidine nécessite pour être efficace une administration prolongée d'au moins 3 jours à des doses très supérieures. De plus, à la différence de la ticlopidine, le clopidogrel peut être administré par voie intraveineuse et présente par cette voie des effets antiagrégants tout à fait équivalents à ceux obtenus par voie orale (Herbert et al., Cardiovasc. Drug Rev., 1993, *11,* 180-198). Ceci n'est pas le cas de la ticlopidine qui ne peut être administrée que par voie orale.

De manière tout à fait surprenante et inattendue, l'association clopidogrel-aspirine de l'invention s'est révélée être pourvue d'une activité synergique des deux principes actifs. Cet effet se caractérise vis à vis de l'agrégation des plaquettes de lapin au collagène, seul agent agrégent pouvant être utilisé en raison de sa dépendance conjointe par l'ADP et par le métabolisme de l'acide arachidonique.

De plus, un effet synergique similaire a été observé vis à vis de la formation d'un thrombus d'origine artérielle induite par l'implantation d'une surface thrombogène (fil de soie) implantée dans un cathéter joignant l'artère carotide et la veine jugulaire du lapin.

Les associations selon l'invention ne majorent pas le risque hémorragique apprécié sur l'allongement du temps de saignement et sont par ailleurs peu toxiques. Leur toxicité est compatible avec leur utilisation comme médicament pour le traitement des troubles et des maladies d'origine thrombotiques citées ci-dessus.

Les associations selon l'invention peuvent être formulés dans des compositions pharmaceutiques pour l'administration aux mammifères, y compris l'homme, pour le traitement des maladies susdites.

Selon l'invention, le clopidogrel et l'aspirine peuvent être administrés sous forme de sel pharmaceutiquement acceptable.

Ces sels sont ceux couramment utilisés en pharmacie tels que les acétate, benzoate, fumarate, maléate, citrate, tartrate, gentisate, méthanesulfonate, éthanesulfonate, benzènesulfonate, laurylsulfonate, dobésilate et paratoluènesulfonate.

Dans la suite, les quantités de clopidogrel et d'aspirine sont exprimées en équivalents de clopidogrel et d'aspirine sous forme libre, non salifiée.

De manière avantageuse, les compositions de l'invention comprennent du clopidogrel et de l'aspirine dans un rapport molaire (aspirine/clopidogrel) compris entre 2,5 et 11,5, de préférence entre 5 et 9, mieux encore entre 7 et 8.

Les associations selon l'invention peuvent être utilisés à des doses journalières de clopidogrel ou d'aspirine de 0,1 à 100 mg par kilo de poids corporel du mammifère à traiter.

Chez l'être humain, la dose peut varier pour chacun des composants de 1 à 500 mg par jour, selon l'âge du sujet à traiter ou le type de traitement : prophylactique ou curatif.

Dans les compositions pharmaceutiques de la présente invention, les principes actifs sont généralement formulés en unités de dosage contenant de 0,1 à 500 mg dudit principe actif par unité de dosage.

La présente invention a donc pour objet les compositions pharmaceutiques qui contiennent à titre de principe actif une association de clopidogrel et d'aspirine. Ces compositions sont préférablement réalisées de façon à pouvoir être administrées par la voie orale ou parentérale.

Dans les compositions pharmaceutiques de la présente invention pour l'administration orale, sublinguale, sous-cutanée, intramusculaire, intraveineuse, transdermique, locale ou rectale, l'ingrédient actif peut être administré sous formes unitaires d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Les principes actifs des associations peuvent être également présenté sous forme de complexe avec une cyclodextrine, par exemple α, β ou γ cyclodextrine, 2-hydroxypropyl-β-cyclodextrine ou méthyl-β-cyclodextrine.

Lorsque les compositions de l'invention sont administrées chez l'homme par voie parentérale et/ou orale, on préfère que la dose journalière de clopidogrel soit comprise entre 50 et 100 mg, la dose journalière d'aspirine étant comprise entre 100 et 500 mg.

On notera que selon l'invention, le clopidogrel et l'aspirine peuvent être tous deux administrés par voie orale, ou bien tous deux par voie parentérale ou bien l'un peut être administré par voie orale (de préférence l'aspirine) et l'autre par voie parentérale (de préférence le clopidogrel).

Selon un mode de réalisation préféré, la dose journalière de clopidogrel administrée chez l'homme par voie parentérale et/ou orale est comprise entre 65 et 100 mg, mieux encore entre 65 et 85 mg, la dose journalière d'aspirine administrée par voie parentérale étant comprise entre 200 et 400 mg, mieux encore entre 315 et 335 mg.

De préférence, la dose de clopidogrel est dans ce cas de 75 mg par jour et la dose d'aspirine est de 325 mg par jour.

Les associations de principe actifs selon l'invention ont fait l'objet d'études pharmacologiques. Des essais ont été réalisés vis à vis du test d'agrégation des plaquettes de lapin au collagène comme décrit précédemment (Born et al., J. Physiol., 1963, *168*, 178-95). Brièvement, des lapins Neo-Zélandais de 2,5 à 3 kg ont été traités par voie orale par de la ticlopidine (100 mg/kg/j) pendant 3 jours ou par voie intraveineuse avec du clopidogrel (10 mg/kg). Une heure après la dernière administration, les animaux ont été traités par voie intraveineuse par l'aspirine (1 mg/kg).

Cinq minutes après l'administration d'aspirine, les animaux ont été anesthésiés à l'éther et 2 ml de sang ont été prélevés à l'artère médiane de l'oreille et mélangés à 0,2 ml d'une solution à 3,8% de citrate de sodium dans l'eau. Le plasma riche en plaquettes a été obtenu par centrifugation du sang à 500g pendant 10 minutes à 15°C. Le nombre de plaquettes a ensuite été ajusté à 106 cellules par µl à l'aide de plasma pauvre en plaquettes obtenu par centrifugation (3000g, 15 min.) du sang anticoagulé.

L'agrégation des plaquettes a été mesurée selon la méthode de Born (Born et al., J. Physiol., 1963, *168*, 178-95) à l'aide d'un agrégomètre à double canal (Chrono Log) sous agitation (900 rpm) à 37°C. L'agrégation des plaquettes a été induite par le collagène (12,5 µg/ml).

L'effet antithrombotique de l'association clopidogrel ou ticlopidine avec l'aspirine a été déterminé vis à vis de la formation d'un thrombus sur un fil de soie présent dans un shunt artério-veineux implanté entre l'artère carotide et la veine jugulaire du lapin comme décrit par Umetsu et al. (Thromb. Haemostas., 1978, *39*, 74-83). Brièvement, des lapins Néo-Zéiandais de 2,5 à 3 kg ont été traités par voie orale par de la ticlopidine (100 mg/kg/j) pendant 3 jours ou par voie intraveineuse avec du clopidogrel (10 mg/kg).

Les animaux ont été anesthésiés par administration sous-cutanée de pentobarbital sodique (30 mg/kg). Deux tubes en polyéthylène longs de 12 cm (diamètre intérieur: 0,6 mm; diamètre extérieur: 0,9 mm) attachés par une partie centrale de 6 cm de long (diamètre intérieur: 0,9 mm) contenant un fil de soie de 5 cm de long ont été placés entre l'artère carotide droite et la veine jugulaire gauche. Une heure après la dernière administration de ticlopidine ou de clopidogrel, les animaux ont été traités par voie intraveineuse par l'aspirine (1 mg/kg). La partie centrale du shunt a ensuite été placée puis retirée après 20 minutes de circulation du sang dans le shunt. Le poids du thrombus présent sur le fil de soie a ensuite été déterminé.

Les résultats montrés dans le TABLEAU 1 indiquent que le clopidogrel (10 mg/kg) ou l'aspirine (1 mg/kg) administrés par voie intraveineuse en dose unique chez le lapin inhibent l'agrégation des plaquettes induite par le collagène. La ticlopidine, administrée par voie orale (100 mg/kg/j) pendant 3 jours présente elle aussi un effet inhibiteur significatif vis à vis de l'agrégation des plaquettes au collagène.

Dans tous les cas, l'administration conjointe de clopidogrel et d'aspirine a résulté en un effet synergique significatif vis à vis de l'agrégation des plaquettes au collagène. C'est à dire que lorsque les produits ont été administrés en association, l'effet antiagrégant obtenu a toujours été supérieur à la simple somme des effets des deux produits testés pris séparément.

Comparativement au simple effet additif observé entre l'effet anti-agrégant de la ticlopidine et de l'aspirine obtenu et revendiqué dans le brevet FR 73 03503, cette activité est tout à fait nouvelle et inattendue.

De la même manière, l'activité antithrombotique du clopidogrel a été potentialisée par une association avec l'aspirine. Dans ces conditions, et comme vis à vis de l'agrégation des plaquettes au collagène, un effet synergique significatif a été observé (TABLEAU 2).

**TABLEAU 1**

| Effet des produits seuls ou en association vis à vis de l'agrégation des plaquettes de lapin au collagène. | | |
|---|---|---|
| Principes actifs | Doses | % d'inhibition |
| Ticlopidine | 100 mg/kg/J - 3 J | 35 ± 3% |
| Clopidogrel | 10 mg/kg | 42 ± 6% |
| Aspirine | 1 mg/kg | 21 ± 2% |
| Ticlopidine + Aspirine | 100 + 1 mg/kg | 52 ± 6% |
| Clopidogrel + Aspirine | 10 + 1 mg/kg | 98 ± 1% |
| Les valeurs indiquées dans le tableau sont des valeurs moyennes sur cinq expériences ± erreurs standards (n=5) | | |

**TABLEAU 2**

| Effet des produits seuls ou en association vis à vis de la formation d'un thrombus artériel sur un fil de soie implanté dans un shunt artério-veineux chez le lapin. | | |
|---|---|---|
| Principes actifs | Doses | % d'inhibition |
| Ticlopidine | 100 mg/kg/J - 3 J | 25 ± 9% |
| Clopidogrel | 10 mg/kg | 34 ± 4% |
| Aspirine | 1 mg/kg | 19 ± 5% |
| Ticlopidine + Aspirine | 100 + 1 mg/kg | 45 ± 3% |
| Clopidogrel + Aspirine | 10 + mg/kg | 82 ± 1% |
| Les valeurs indiquées dans le tableau sont des valeurs moyennes sur cinq expériences ± erreurs standards (n = 5) | | |

## Revendications

1. Composition pharmaceutique contenant une association de principes actifs dans lequel les principes actifs sont le clopidogrel et l'aspirine les deux constituants étant présent à l'état libre ou sous forme de sel pharmaceutiquement acceptable.

2. Composition pharmaceutique contenant une association de principes actifs selon la revendication 1 en association avec au moins un excipient pharmaceutique.

3. Composition pharmaceutique selon l'une quelconque des revendications 1 ou 2 sous une forme administrable par voie parentérale ou par voie orale.

4. Composition pharmaceutique selon l'une quelconque des revendications précédentes, **caractérisée en ce que** le clopidogrel et l'aspirine sont présents dans un rapport molaire aspirine/clopidogrel compris entre 2,5 et 11,5, de préférence entre 5 et 9.

5. Composition pharmaceutique selon l'une quelconque des revendications précédentes pour le traitement d'une pathologie induite par l'agrégation plaquettaire incluant l'angor stable ou instable, les troubles du système cardiovasculaire et cérébrovasculaire comme les troubles thromboemboliques associées à l'athérosclérose et au diabète tels l'angine instable, l'attaque cérébrale, la resténose après angioplastie, l'endartérectomie ou pose de prothèses endovasculaires métalliques ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires, de pontages aorto coronariens.

6. Utilisation d'une composition selon l'une quelconque des revendications précédentes pour la préparation d'un médicament destiné au traitement d'une pathologie induite par l'agrégation plaquettaire incluant l'angor stable ou instable, les troubles du système cardiovasculaire et cérébrovasculaire comme les troubles thromboemboliques associées à l'athérosclérose et au diabète tels l'angine instable, l'attaque cérébrale, la resténose après angioplastie, l'endartérectomie ou pose de prothèses endovasculaires métalliques ou les troubles thromboemboliques associés à la rethrombose après thrombolyse, à l'infarctus, à la démence d'origine ischémique, aux maladies artérielles périphériques, à l'hémodialyse, aux fibrillations auriculaires ou encore lors de l'utilisation de prothèses vasculaires, de pontages aorto coronariens, ledit traitement impliquant l'administration chez l'homme de 1 à 500 mg par jour de clopidogrel et de 1 à 500 mg par jour d'aspirine, les doses étant exprimées en quantité équivalente de clopidogrel et d'aspirine sous forme libre.

7. Utilisation selon la revendication 6, dans laquelle le traitement implique l'administration par voie parentérale et/ou orale de 50 à 100 mg de clopidogrel par jour et de 100 à 500 mg d'aspirine par jour.

8. Utilisation selon la revendication 6, dans laquelle le traitement implique l'administration par voie parentérale et/ou orale de 65 à 100 mg, de préfence de 65 à 85 mg de clopidogrel par jour et de 200 à 400 mg, de préférence de 315 à 335 mg d'aspirine par jour.

## Patentansprüche

1. Pharmazeutische Zubereitung enthaltend eine Wirkstoffkombination, in der die Wirkstoffe Clopidogrel und Aspirin sind und die beiden Bestandteile in freier Form oder in Form des pharmazeutisch annehmbaren Salzes vorliegen.

2. Pharmazeutische Zubereitung enthaltend eine Wirkstoffkombination nach Anspruch 1 in Kombination mit mindestens einem pharmazeutischen Trägermaterial.

3. Pharmazeutische Zubereitung nach irgendeinem der Ansprüche 1 oder 2 in einer zur Verabreichung auf parenteralem oder oralem Wege geeigneten Form.

4. Pharmazeutische Zubereitung nach irgendeinem der vorhergehenden Ansprüche, **dadurch gekennzeichnet, daß** Clopidogrel und Aspirin in einem Aspirin/Clopidogrel-Molverhältnis zwischen 2,5 und 11.5, vorzugsweise zwischen 5 und 9 vorhanden sind.

5. Pharmazeutische Zubereitung nach irgendeinem der vorhergehenden Ansprüche zur Behandlung eines pathologischen Zustandes, der verursacht worden ist durch die Plättchenaggregation, einschließlich stabilem oder instabilem Angor, Störungen des cardiovaskulären und cerebrovaskulären Systems, wie thromboembolische Störungen, die mit der Atherosklerose und dem Diabetes verbunden sind, wie instabile Angina, Cerebralvonälle, Restenose nach der Angioplastie, der Endarterektomie oder der Anordnung von metallischen endovaskulären Prothesen, oder von thromboembolischen Störungen, die mit der Rethrombose nach der Thrombolyse, dem Infarkt, der Demenz ischämischen Ursprungs. peripheren arteriellen Erkrankungen, der Hämodialyse oder Kammerflimmern verknüpft sind, oder bei der Verwendung von Gefäßprothesen und Koronaraortabrücken (Bypassen).

6. Verwendung einer Zubereitung nach irgendeinem der vorhergehenden Ansprüche für die Herstellung eines Arzneimittels zur Behandlung eines pathologischen Zustandes, der verursacht worden ist durch die Plättchenaggregation, einschließlich stabilem oder instabilem Angor, Störungen des cardiovaskulären und cerebrovaskulären Systems, wie thromboemboltsche Störungen, die mit der Atherosklerose und dem Diabetes verbunden sind, wie instabile Angina, Cerebralvorfälle, Restenose nach der Angioplastie, der Endarterektomie oder der Anordnung von metallischen endovaskulären Prothesen, oder von thromboembolischen Störungen, die mit der Rethrombose nach der Thrombolyse, dem Infarkt, der Demenz ischämischen Ursprungs, peripheren arteriellen Erkrankungen, der Hämodialyse oder Kammerflimmern verknüpft sind, oder bei der Verwendung von Gefäßprothesen und Koronaraortabrücken (Bypassen), wobei die Behandlung die tägliche Verabreichung von 1 bis 500 mg Clopidogrel und 1 bis 500 mg Aspirin an den Menschen umfaßt und die Dosierungen als Menge angegeben ist, die Clopidogrel und Aspirin in freier Form äquivalent ist.

7. Verwendung nach Anspruch 6, worin die Behandlung die tägliche Verabreichung auf parenteralem und/oder oralem Wege von 50 bis 100 mg Clopidogrel und 100 bis 500 mg Aspirin umfaßt.

8. Verwendung nach Anspruch 6. worin die Behandlung die tägliche Verabreichung auf parenteralem und/oder oralem Wege von 65 bis 100, vorzugsweise 65 bis 85 mg Clopidogrel und 200 bis 400 mg, vorzugsweise 315 bis 335 mg Aspirin umfaßt.

## Claims

1. Pharmaceutical composition containing a combination of active substances in which the active substances are clopidogrel and aspirin, the two ingredients being present in free form or in the form of pharmaceutically acceptable salts.

2. Pharmaceutical composition containing a combination of active substances according to claim 1 combined with at least one pharmaceutical excipient.

3. Pharmaceutical composition according to either of claims 1 and 2 in a form which can be administered by parenteral route or by oral route.

4. Pharmaceutical composition according to any one of the preceding claims, **characterised in that** the clopidogrel and the aspirin are present in a molar ratio of aspirin/clopidogrel of between 2.5 and 11.5, preferably between 5 and 9.

5. Pharmaceutical composition according to any one of the preceding claims for the treatment of an illness caused by platelet aggregation including stable or unstable angina, disorders of the cardiovascular and cerebrovascular system such as thromboembolic disorders associated with atherosclerosis and diabetes such as unstable angina, stroke, restenosis after angioplasty, endarterectomy or the fitting of metal endovascular prostheses or thromboembolic disorders associated with rethrombosis after thrombolysis, infarct, dementia of ischaemic origin, peripheral arterial disease, haemodialysis, ventricular fibrillations or in the use of vascular prostheses and aorto-coronary bridges.

6. Use of a composition according to any one of the preceding claims for the preparation of a medicament intended to treat an illness caused by platelet aggregation including stable or unstable angina, disorders of the cardiovascular and cerebrovascular system such as thromboembolic disorders associated with atherosclerosis and diabetes such as unstable angina, stroke, restenosis after angioplasty, endarterectomy or the fitting of metal endovascular prostheses or thromboembolic disorders associated with rethrombosis after thrombolysis, infarct, dementia of ischaemic origin, peripheral arterial disease, haemodialysis, ventricular fibrillations or in the use of vascular prostheses and aorto-coronary bridges, said treatment involving administering to humans 1 to 500 mg per day of clopidogrel and 1 to 500 mg per day of aspirin, the doses being expressed as the equivalent amount of clopidogrel and aspirin in free form.

7. Use according to claim 6, wherein the treatment involves the administration by parenteral and/or oral route of 50 to 100 mg of clopidogrel per day and 100 to 500 mg of aspirin per day.

8. Use according to claim 6, wherein the treatment involves the administration by parenteral and/or oral route of 65 to 100 mg, preferably 65 to 85 mg of clopidogrel per day and 200 to 400 mg, preferably 315 to 335 mg of aspirin per day.
